# EUROPÄISCHE PATENTANMELDUNG

(11) **EP 1 797 862 A1**
(43) Veröffentlichungstag der Anmeldung: **20.06.2007**
(21) Anmeldenummer: 06025296.2
(22) Anmeldetag: 07.12.2006
(51) Int. Cl.: A61K 8/39, A61K 8/02, A61Q 5/02, A61Q 19/10, C11D 17/04, C11D 1/08

(54) **Reinigungstücher**

(30) Priorität: 16.12.2005 DE 102005060733
(71) Anmelder: Cognis IP Management GmbH, 40589 Düsseldorf (DE)
(72) Erfinder: Eisfeld, Wolf, 40597 Düsseldorf (DE); Dickhof, Susanne, 41748 Viersen (DE); Behler, Ansgar, 46240 Bottrop (DE); Issberner, Ulrich, 19002 Ambler (US); Küsters, Esther, 40229 Düsseldorf (DE); Marten, Sandra, 40597 Düsseldorf (DE)

(57) **Zusammenfassung**

Vorgeschlagen werden Reinigungstücher, die dadurch gekennzeichnet sind, dass ein wasserunlösliches Substrat mit einem Alkylethercitrat imprägniert ist.

## Beschreibung

### Gebiet der Erfindung

Die Erfindung befindet sich auf dem Gebiet der trockenen und feuchten Reinigungstücher, sowie ihrer Anwendung bei der Reinigung und Pflege von Haut und Haaren.

### Stand der Technik

Feuchte oder trockene Reinigungstücher erfreuen sich immer größerer Popularität und Bedeutung auf dem Gebiet der Kosmetik. Neben lange bekannten Küchentüchern, Toilettenpapieren, Papiertaschentüchern, Gesichtstüchern und -pads, Erfrischungstüchern u.ä. existieren auch feuchte Reinigungstücher, die häufig auch als wet wipes bezeichnet werden. Feuchte Reinigungstücher sind textile Flächengebilde verschiedenster Ausführungsformen (Vlies, Tissue oder Papier), die mit einer Lösung imprägniert sind. Aus US 5972361, US 6063397, US 5980931 und WO 98/18446 sind trockene oder feuchte Reinigungstücher für die Reinigung von Haut und Haar bekannt, die mit Lösungen enthaltend Tenside und Konditioniermittel imprägniert sind.

Eine andere Form der Haarreinigung besteht in der Anwendung von wasserfreien Reinigungspudern, die auf den Haaren befindliche Fette und Schmutzbestandteile binden und anschließend mit einem Handtuch oder durch Bürsten aus dem Haar entfernt werden. Diese Art der Haarreinigung ist sehr beliebt, wenn dafür wenig Zeit oder keine ausreichende Wassermenge zur Verfügung steht - wie z.B. während der Reise. Dies führt jedoch häufig dazu, dass die Haare anschließend nicht nur stumpf und gräulich aussehen, sondern auch einen unangenehmen Griff aufweisen, insbesondere bei langen Haaren.

Die Aufgabe der vorliegenden Erfindung hat daher darin bestanden, Reinigungstücher zur Verfügung zu stellen, die die sensorischen Eigenschaften von Haut und Haar positiv beeinflussen. So sollen die Haare nach einer Reinigung mit den erfindungsgemäßen Reinigungtüchern ein erhöhtes Volumen und dabei keine Verfärbungen aufweisen. Dennoch sollen die erfindungsgemäßen Reinigungtücher über eine hohe Reinigungswirkung bei einfacher und schneller Anwendung haben.

### Beschreibung der Erfindung

Gegenstand der vorliegenden Erfindung sind Reinigungstücher, bei denen ein wasserunlösliches Substrat mit einem Alkylethercitrat imprägniert ist. Überraschenderweise wurde gefunden, dass derartige Reinigungstücher über hervorragende reinigende Eigenschaften verfügen, wobei gleichzeitig Haut und Haare konditioniert werden. Die Reinigungstücher hinterlassen auf Haut und Haaren keine diese verfärbenden Ablagerungen und verbessern Haarvolumen und -glanz. Die erfindungsgemäßen Reinigungstücher können dabei im trockenen Zustand verwendet oder aber auch im Wasser vor Gebrauch angefeuchtet werden.

### Alkylethercitrate

Durch die Verwendung von Alkylethercitraten auf Flächengebilden wird neben der Reinigung gleichzeitig eine Konditionierung von Haut und Haaren erreicht. Haut und Haar weisen anschließend eine höhere Weichheit auf, die Haare zeichnen sich durch erhöhtes Volumen aus. Dies führt zu einem verbesserten taktilen Gefühl und zu einem verbesserten Pflegeeffekt. Mit den erfindungsgemäßen Reinigungstüchern gereinigte Haut oder Haare weisen weniger Rückstände des Reinigungsmittels auf und sind so weniger mit Fremdstoffen beschwert.

Die erfindungsgemäßen Alkylethercitrate leiten sich ab von ethoxylierten Alkoholen der allgemeinen Formel (I)

R¹O(CH₂CH₂O)nH (I),

in der R¹ für einen Alkylrest und n für den Ethoxylierungsgrad steht. Bevorzugt steht R¹ für einen linearen Alkylrest einer Fettalkoholmischung enthaltend 45 - 75 Gew.% C12-, 15 bis 35 Gew.% C14-, 0 bis 15 Gew.% C16- und 0 bis 20 Gew.% C18- Alkohol und n für Zahlen von 5 bis 9, mit der Maßgabe, dass in den Alkylethercitraten das Gewichtsverhältnis von Monoester : Diester im Bereich von 3: 1 bis 10 : 1 liegt.

Bei den Alkoholmischungen handelt es sich um Mischungen hauptsächlich von Caprinalkohol, Laurylalkohol, Myristylalkohol, Cetylalkohol und/oder Stearylalkohol in den angegebenen Gewichtsverhältnissen. Die Mischungen sind entweder durch Mischung der einzelnen Alkohole zugänglich oder durch Abmischung entsprechender Alkoholmischungen. Einer Ausführungsform der vorliegenden Erfindung entsprechend werden Alkylethercitrate von Alkoholen der Formel (I) bevorzugt, wobei R¹ für einen linearen Alkylrest steht, abgeleitet von einer Fettalkoholmischung enthaltend 65 bis 75 Gew.% C12-, 20 bis 30 Gew.% C14-, 0 bis 5 Gew.% C16- und 0 bis 5 Gew.% C18- Alkohol. Bei diesen den Alkylethercitraten zugrunde liegenden Alkoholmischungen handelt es sich um kommerziell erhältliche Alkoholmischungen, beispielsweise um Dehydol LS ^{™}, ein Handelsprodukt der Cognis Deutschland GmbH & Co. KG. Die Fettalkoholmischung weist folgende Kettenverteilung auf in Gew.%: C10: 0 bis 2; C12: 70 bis 75; C14: 24 bis 30; C16: 0 bis 2 und ist beispielsweise aus Palmkern- oder Kokosöl zugänglich.

Einer weiteren Ausführungsform der vorliegenden Erfindung entsprechend werden Alkylethercitrate von ethoxylierten Alkoholen der Formel (I) bevorzugt, in der R¹ für einen linearen Alkylrest steht, abgeleitet von einer Fettalkoholmischung enthaltend 45 bis 60 Gew.% C12-, 15 bis 30 Gew.% C 14-, 5 bis 15 Gew.% C16- und 8 bis 20 Gew.% C 18-Alkohol. Bei diesen den Alkylethercitraten zugrunde liegenden Alkoholmischungen handelt es sich um kommerziell erhältliche Alkoholmischungen, beispielsweise um Dehydol LT ^{™}, ein Handelsprodukt der Cognis Deutschland GmbH & Co. KG. Die Fettalkoholmischung weist folgende Kettenverteilung auf in Gew.% : < C12: 0 bis 3; C12: 48 bis 58; C14: 18 bis 24; C16: 8 bis 12; C18: 11 bis 15; > C18: 0 bis 1% und ist beispielsweise aus Palmkern- oder Kokosöl zugänglich.

Bevorzugt im Sinne der Erfindung ist der Ethoxylierungsgrad n eine Zahl von 6 bis 8, wobei die Zahl ganz oder gebrochen sein kann.

Besonders vorteilhaft sind Ethoxlierungsprodukte von Fettalkoholmischungen enthaltend 45 bis 60 Gew.% C12-, 15 bis 30 Gew.% C14-, 5 bis 15 Gew.% C16- und 8 bis 20 Gew.% C18- Alkohol mit 6 bis 8 Mol Ethylenoxid und insbesondere das Ethoxylierungsprodukt von Dehydol LT ^{™} mit 7 Mol Ethylenoxid.

Bei den (Fett)Alkoholmischungen können noch geringe Mengen an kurzkettigen oder längerkettigen Alkoholen enthalten sein, vorzugsweise unter 10, insbesondere 5 Gew-% in Summe bezogen auf Alkoholmischungen.

Die erfindungsgemäßen Alkylethercitrate sind Mischungen von isomeren Verbindungen der allgemeinen Formel (II) 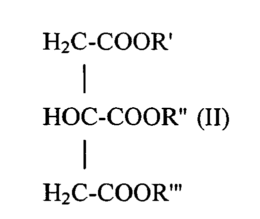 in der R', R", R''' für X und/oder einen ethoxylierten Alkylrest R1 mit der in Formel (I) angegebenen Bedeutung steht, wobei die Verteilung der Reste R', R" bzw. R''' mit der Maßgabe erfolgt, dass das Gewichtsverhältnis von Monoester: Diester im Bereich von 3: 1 bis 10 : 1 liegt, vorzugsweise liegt das Gewichtsverhältnis von Monoester: Diester im Bereich von 5 : 1 bis 8 : 1.

Die erfindungsgemäßen Alkylethercitrate enthalten somit zwingend Mono- und Diester, vorzugsweise in Mengen von 50 bis 90 Gew.%, insbesondere von 60 bis 80 Gew.% - berechnet als Mono- und Diester und bezogen auf Mischung). Die Mischungen können als den zu 100 Gew.% fehlenden Rest noch Triester und freie Citronensäure enthalten. Vorzugsweise enthalten die Mischungen aber wenig freie Citronensäure, wobei weniger als 10 Gew.% - bezogen auf Mischungen - bevorzugt sind.

Somit stellen die erfindungsgemäßen Alkylethercitrate hauptsächlich Partialester der Citronensäure dar, die noch mindestens eine freie Carboxylgruppe enthalten. Dementsprechend kann es sich auch um saure Ester oder deren Neutralisationsprodukte handeln, und X kann in Formel (II) für Wasserstoff oder ein Kation stehen. Vorzugsweise liegen die Partialester dann in Form von Alkali-, Erdalkali-, Ammonium-, Alkylammonium-, Alkanolammonium- und/oder Glucammoniumsalze vor (d.h. X steht für Alkali-, Erdalkali-, Ammonium-, Alkylammonium-, Alkanolammonium- und/oder Glucammonium-Ion).

Die erfindungsgemäß zu verwendenen Alkylethercitrate zeichnen sich insbesondere durch ihre Eigenschaft aus, das Aufziehen von Alkylethersulfaten auf Haut und Haar zu beschränken und sind daher als milde Tenside bekannt.

### wasserunlösliche Substrate

Als wasserunlösliche Substrate eigenen sich ein- oder mehrlagig aufgebaute Flächengebilde. Neben papierbasierten Tissues kommen auch entsprechende Tissuegewebe in Frage, die aus Faser- oder Vliesstoff hergestellt werden. Zu Beispielen für Naturfasern zählen Seide, Cellulose, Keratin, Wolle, Baumwolle, Jute, Leinen, Flachs, für synthetische Fasern Acetat-, Acrylat-, Celluloseester-, Polyamid-, Polyester-, Polyolefin-, Polyvinylalkohol-, Polyurethan- Fasern, oder auch durch Additive hydrophilierte Polyolefingewebe sowie Mischungen dieser Fasern bzw. Gewebe. Umsetzungsprodukte von 1 Teil Polyethylenglykol mit 2 Teilen Fettsäuren mit 10 bis 12 C-Atomen oder deren Derivaten werden hierbei zur Hydrophilierung der Polyolefin-haltigen Gewebe eingesetzt.
Bevorzugt sind nicht-gewebte Stoffe, da diese besser mit der erfindungsgemäßen erwünschten Struktur versehen werden können, dazu eignen sich Träger aus Viskose- Polyester-Mischungen besonders. Bevorzugt sind jedoch durch Wassereinwirkung vernetzte Trägersysteme von 50 bis 90 Gew. % Viskose und 50 bis 10 Gew. % Polyester, besonders bevorzugt sind Träger aus 60 bis 80 Gew. % Viskose und 40 bis 20 Gew. % Polyester, speziell werden Stoffe mit 65 bis 70 Gew. % Viskose und 35 bis 30 Gew. % Polyester eingesetzt.

Die Größe der Tücher liegt in der Regel zwischen 100 und 500 mm in der Länge und zwischen 100 und 500 mm in der Breite, wobei Längen- und Breitenmaße zwischen 120 und 220 mm bevorzugt sind. Das Gewebe kann aber auch in Handschuhform vorliegen und ist dann möglicherweise mehrlagig, so dass die innere Gewebelage des Handschuhs hydrophober ausfällt, eine Barrierefunktion hat und einen Schutz vor der Berührung der Hand mit der Formulierung oder mit Feuchtigkeit bietet.

Die Trägerstoffe der erfindungsgemäßen Kosmetiktücher weisen auf Grund ihrer Herstellung - z.B. durch Hydroentangling oder Meltbonding - eine gleichmäßig, strukturierte Oberfläche mit punktförmigen bis ovalen Vertiefungen auf. Diese Vertiefungen - auch Grübchen genannt - haben eine runde bis ovale Form mit 0,1 bis 1 mm, vorzugsweise 0,2 bis 0,6 mm Durchmesser resp. Breite und 0,5 bis 5,0 mm, vorzugsweise 0,8 bis 1,5 mm Durchmesser resp. Länge. Sie können beidseitig vorliegen oder nur von einer Seite. Bei einseitigen Vertiefungen nehmen sie zwischen 50 und 99 %, vorzugsweise zwischen 60 und 85 % der Dicke des Trägers ein, bei beidseitigen Vertiefungen muß dieser Anteil entsprechend aufgeteilt werden. Pro 100 mm² Trägerfläche liegen im Durchschnitt zwischen 500 und 4000, vorzugsweise zwischen 1500 und 3500, und besonders bevorzugt zwischen 2500 und 3200 solcher Grübchen vor.
In einer weiteren Ausführungsform sind die Flächengebilde neben den Alkylethercitraten noch mit mindestens einem weiteren Tensid imprägniert. Hierzu können bekannte anionische, kationische, nichtionische, zwitterionische und/oder amphotere Tenside verwendet werden.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Alkyloligoglucosidcarboxylate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen.

Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

In einer besonders bevorzugten Ausführungsform werden Kombinationen von Alkylethercitraten mit acylierten Aminosäuren - insbesondere Acylglutamaten - und/oder Alkyloligoglucosidcarboxylaten eingesetzt.

Die Gesamttensidmenge sollte sich erfindungsgemäß zwischen 0,1 und 10 Gew.% in bezug auf die Gesamtzusammensetzung bewegen. Tensidmengen oberhalb von 10 Gew.% führen zu einem Aufziehen der Tenside auf Haut und Haar, was unerwünscht ist. Besonders bevorzugt sind Tensidgehalte zwischen 1 und 5 Gew.% in bezug auf die Gesamtzusammensetzung.

### Entfettungsmittel

Weiterhin können die erfindungsgemäßen Reinigungstücher - insbesondere für die Haarreinigung - mit einem Entfettungsmittel imprägniert sein. Dabei kommen insbesondere niedere lineare oder verzweigte Alkohole und besonders bevorzugt Ethanol oder Isopropanol zum Einsatz.

### Moisturizer

Die erfindungsgemäßen Reinigungstücher können weiterhin zusätzlich mit einem Moisturizer ausgerüstet sein. Hier sind insbesondere polyhydrische Alkohole mit 1 bis 3 Kohlenstoffatomen zu nennen, wie z.B. Propylenglykol und Glycerin, die im Sinne der vorliegenden Erfindung besonders bevorzugt sind.

### Wasser und andere Hilfsstoffe

Die erfindungsgemäßen Reinigungstücher können mit wasserfreien oder wasserarmen Reinigungszubereitungen imprägniert sein, die entweder direkt mit geringem Wassergehalt auf die Tücher imprägniert oder die in wässriger Lösung aufgebracht und anschließen einem Trocknungsschritt unterworfen werden. Die zur Imprägnierung der Reinigungstücher verwendeten Zusammensetzungen können neben den bereits oben aufgeführten Inhaltsstoffen weitere Ölkörper, Wachse, Perlglanz- und Trübungsmittel, Emulgatoren, Verdickungsmittel, Polymere, Überfettungsmittel, Stabilisatoren, Silikonverbindungen, Biogene Wirkstoffe, Deodorantien und keimhemmende Mittel, Antitranspirantien, Filmbildner, Antischuppenwirkstoffe, Quellmittel, Insektenrepellentien, Selbstbräuner, Depigmentierungsmittel, Hydrotrope, Konservierungsmittel, UV-Schutzmittel, Parumöle, Aromen oder Farbstoffe enthalten.

Die erfindungsgemäßen Reinigungstücher können als Haar- oder Hautreinigungstücher oder als Reinigungstuch zur Reinigung und Pflege von Haus- und Nutztieren verwendet werden.

### Beispiele

### Beispiel 1: Formulierung zur Imprägnierung von Reinigungstüchern (Mengenangaben als Aktivsubstanz in Gew.%)

| | |
|---|---|
| Plantapon LC 7 | 1% |
| Plantapon S | 0.75% |
| Plantapon ACG 35 | 0.25% |
| Ethanol | 10% |
| Propylene Glycol | 3% |
| Water | ad 100% |

### Beispiel 2: Formulierung zur Imprägnierung von Reinigungstüchern (Mengenangaben als Aktivsubstanz in Gew.%)

| | |
|---|---|
| Plantapon LC7 | 2% |
| Ethanol | 10% |
| Propylene Glycol | 3% |
| Water | 85% |

### Beispiel 3: Formulierung zur Imprägnierung von Reinigungstüchern (Mengenangaben als Aktivsubstanz in Gew.%)

| | |
|---|---|
| Plantapon LC7 | 13,34% |
| Ethanol | 66,66% |
| Propylene Glycol | 20% |

Das o.g. Konzentrat wird vor Imprägnierung der Reinigungstücher mit Wasser in einem Verhältnis von 3 : 17 (1 Liter Konzentrat + 5,67 Liter Wasser) vermischt.

### Beispiel 4: Formulierung zur Imprägnierung von Reinigungstüchern (Mengenangaben als Aktivsubstanz in Gew.%)

| | |
|---|---|
| Plantapon LC7 | 1% |
| Plantapon LGC | 1% |
| Ethanol | 10% |
| Propylene Glycol | 3% |
| Water | 85% |

**Beispiel 5 :** In dieser Tabelle sind Rezepturen aufgeführt, die aufgrund der Scherempfindlichkeit und/oder der niedrigen Viskosität sprühbar sind und sich dadurch besonders für eine Anwendung für Tissues, Papiere, Wipes, Schwämme, Pflaster im Bereich Baby-Hygiene, Baby-Pflege, Hautpflege, Reinigung, Gesichtsreinigung, eignen.
Die Angaben sind Gew.%:

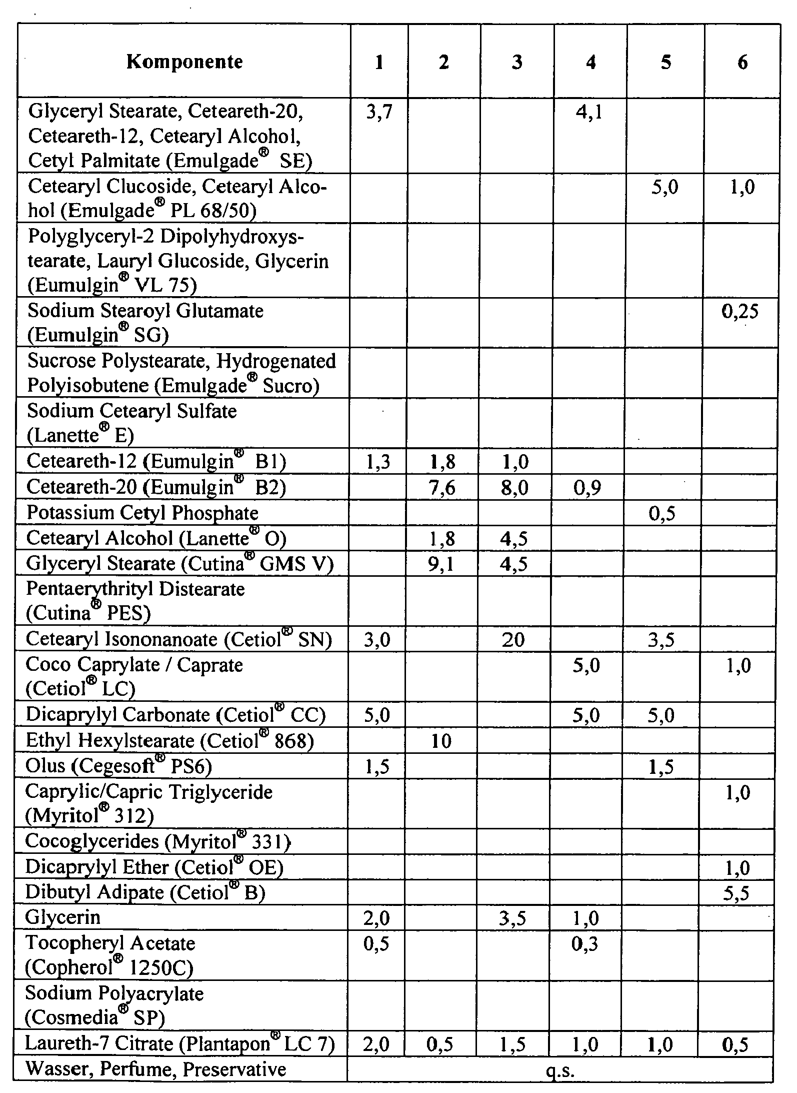

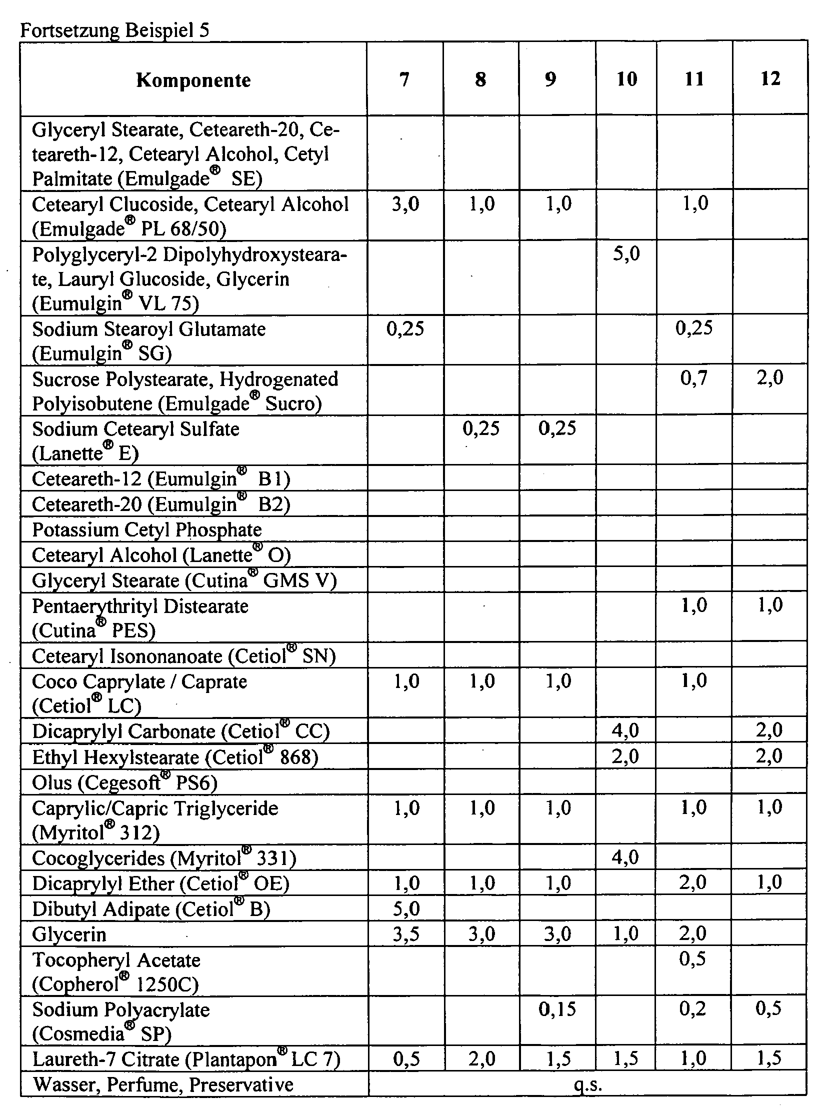

## Patentansprüche

1. Reinigungstücher, **dadurch gekennzeichnet, dass** ein wasserunlösliches Substrat mit einem Alkylethercitrat imprägniert ist.

2. Reinigungstücher gemäß Anspruch 1, **dadurch gekennzeichnet, dass** sie mit mindestens einem weiteren Tensid imprägniert sind.

3. Reinigungstücher nach einem der Ansprüche 1 und/oder 2, **dadurch** gekennzeichnt, dass sie weiterhin mit einem Entfettungsmittel imprägniert sind.

4. Reinigungstücher nach wenigstens einem der Ansprüche 1 bis 3, **dadurch gekennzeichnet, dass** sie weiterhin mit einem Moisturizer imprägniert sind.

5. Reinigungstücher nach wenigstens einem der Ansprüche 1 bis 4, **dadurch gekennzeichnet, dass** das Entfettungsmittel ausgewählt ist aus der Gruppe, die gebildet wird von Ethanol und Isopropanol oder Mischungen hiervon.

6. Reinigungstücher nach wenigstens einem der Ansprüche 1 bis 5, **dadurch gekennzeichnet, dass** der Moisturizer ausgewählt ist aus der Gruppe, die gebildet wird von polyhydrischen Alkoholen mit 1 bis 3 Kohlenstoffatomen.

7. Verwendung der Reinungstücher gemäß wenigstens einem der Ansprüche 1 bis 6 als Haarreinigungstuch.

8. Verwendung der Reinungstücher gemäß wenigstens einem der Ansprüche 1 bis 6 als Hautreinigungstuch.

9. Verwendung der Reinungstücher gemäß wenigstens einem der Ansprüche 1 bis 6 als Reinigungstuch zur Reinigung und Pflege von Haus- und Nutztieren.
